# EUROPEAN PATENT SPECIFICATION

(11) **EP 3 826 686 B1**
(45) Date of publication and mention of the grant of the patent: **21.05.2025**
(21) Application number: 18783136.7
(22) Date of filing: 25.09.2018
(51) Int. Cl.: A61K 51/08, A61K 51/12, A61K 47/22, A61K 47/12, A61K 9/08, A61K 9/00

(54) **STABLE, CONCENTRATED RADIONUCLIDE COMPLEX SOLUTIONS**
STABILE KONZENTRIERTE RADIONUKLID-KOMPLEX-LÖSUNGEN
SOLUTIONS COMPLEXES CONCENTRÉES STABLES DE RADIONUCLÉIDES

(30) Priority: 25.07.2018 WO PCT/IB2018/055575; 25.07.2018 US 201816045484
(43) Date of publication of application: 02.06.2021
(60) Divisional application: 25162390.6
(73) Proprietor: Advanced Accelerator Applications S.A., 01630 Saint Genis Pouilly (FR)
(72) Inventor: CHICCO, Daniela, 86077 Pozzilli Isernia (IT); BARBATO, Donato, 86077 Pozzilli Isernia (IT); DE PALO, Francesco, 86077 Pozzilli Isernia (IT); FUGAZZA, Lorenza, 86077 Pozzilli Isernia (IT); MARIANI, Maurizio, 86077 Pozzilli Isernia (IT); TESORIERE, Giovanni, 86077 Pozzilli Isernia (IT); BRAMBATI, Clementina, 86077 Pozzilli Isernia (IT)
(74) Representative: Carpmaels & Ransford LLP
(86) International application number: PCT/IB2018/057415
(87) International publication number: WO 2020/021322

(56) References cited:
- WO-A1-2008/009444
- DE BARBOZA M ET AL: "PRODUCTION and QUALITY CONTROL of 177 Lu-DOTATATE [ 177 Lu-DOTA-Tyr3]-OCTREOTATE: CLINICAL APPLICATION", 31 December 2009 (2009-12-31), XP093192831, Retrieved from the Internet <URL:https://inis.iaea.org/collection/NCLCollectionStore/_Public/43/004/43004240.pdf>
- DE BARBOZA M ET AL: "PRODUCTION AND QUALITY CONTROL OF 177 LU-DOTATATE [ 177 LU-DOTA-TRY3]-OCTREOTATE: CLINICAL APPLICATION", 31 December 2009 (2009-12-31), XP093192833, Retrieved from the Internet <URL:https://inis.iaea.org/collection/NCLCollectionStore/_Public/43/005/43005461.pdf>
- EMA: "Assessment report: Lutathera", 17 January 2018 (2018-01-17), XP093192834, Retrieved from the Internet <URL:https://www.ema.europa.eu/en/documents/assessment-report/lutathera-epar-public-assessment-report_en.pdf>
- ANUPAM MATHUR ET AL: "Bulk Scale Formulation of Therapeutic Doses of Clinical Grade Ready-to-Use 177 Lu-DOTA-TATE: The Intricate Radiochemistry Aspects", CANCER BIOTHERAPY & RADIOPHARMACEUTICALS, vol. 32, no. 7, 1 September 2017 (2017-09-01), US, pages 266 - 273, XP055558673, ISSN: 1084-9785, DOI: 10.1089/cbr.2017.2208
- STEPHAN MAUS ET AL: "Aspects on radiolabeling of 177Lu-DOTA-TATE: After C18 purification re-addition of ascorbic acid is required to maintain radiochemical purity", INTERNATIONAL JOURNAL OF DIAGNOSTIC IMAGING, SCIEDU PRESS, CA, vol. 1, no. 1, 1 March 2014 (2014-03-01), pages 5 - 11, XP002738789, ISSN: 2331-5857, [retrieved on 20140221], DOI: 10.5430/IJDI.V1N1P5
- LUNA-GUTIÉRREZ MYRNA ET AL: "Freeze-dried multi-dose kits for the fast preparation of177Lu-Tyr3-octreotide and177Lu-PSMA(inhibitor) under GMP conditions", JOURNAL OF RADIOANALYTICAL AND NUCLEAR CHEMISTRY, AKADEMIAI KIADO RT, HU, vol. 314, no. 3, 2 November 2017 (2017-11-02), pages 2181 - 2188, XP036380439, ISSN: 0236-5731, [retrieved on 20171102], DOI: 10.1007/S10967-017-5595-1

## Description

### FIELD OF THE INVENTION

The present invention relates to a process for manufacturing radionuclide complex solutions of high concentration and of high chemical and radiochemical stability, that allows their use as commercial drug product for diagnostic and/or therapeutic purposes.

### BACKGROUND OF THE INVENTION

The concept of targeted drug delivery is based on cell receptors which are overexpressed in the target cell in contrast to the not-to-be-targeted cells. If a drug has a binding site to those overexpressed cell receptors it allows the delivery of the drug after its systemic administration in high concentration to those target cells while leaving other cells, which are not of interested, unaffected. For example, if tumor cells are characterized by an overexpression of a specific cell receptor, a drug with binding affinity to said receptor will after intravenous infusion accumulate in high concentration in the tumor tissue while leaving the normal tissue unaffected.

This targeted drug delivery concept has also been used in radiomedicine to deliver radionuclides selectively to the target cells for diagnostic or therapeutic purposes.

For this radiomedicinal application the target cell receptor binding moiety is typically linked to a chelating agent which is able to form a strong complex with the metal ions of a radionuclide. This radiopharmaceutical drug is then delivered to the target cell and the decay of the radionuclide is then releasing high energy electrons, positrons or alpha particles as well as gamma rays at the target site.

One technical problem with those radiopharmaceutical drug products is that the decay of the radionuclide occurs constantly, e.g. also during the manufacturing and during storage of the drug product, and the released high energy emissions induce the cleavage of the chemical bonds of the molecules which form part of the drug product. This is often referred to as radiolysis or radiolytic degradation. The radiolytic degradation of the receptor binding moiety of the drug may lead to a decrease in its efficacy to act as a diagnostic and/or therapeutic.

The poor stability of those radiopharmaceutical drug products and their lack of any significant shelf-life required that those drugs have so far to be manufactured as an individual patient's dose unit in the laboratories at the hospital and administered immediately to the patient who had to be present at that hospital already awaiting the radiological treatment. To facilitate such drug preparation in the hospital laboratories, "cold" (i.e. non-radioactive) freeze-dried kits have been developed which comprise the cell receptor binding moiety linked to a chelating agent without the radionuclide. The freeze-dried content of those kit vials is then to be reconstituted with an solution of the radionuclide short before administration (Das et al. J Radioanal Nucl Chem 2014, 299, 1389-1398; Das et al. Current Radiopharmaceuticals 2014, 7, 12-19*;* Luna-Gutierrez et al. J Radioanal Nucl Chem 2017, 314, 2181-2188). However, those kits are not "ready-to-use" as they require the reconstitution step and in addition further processing steps (e.g. applying heat for the complexation reaction) as well as purification and sterilization steps before the drug can be finally administered.

To reduce radiolysis of radiopharmaceutical drug products and thus improve stability, various strategies have been explored with more or less success: The drug product may be stored at low temperatures, or produced in high dilution, or stabilizers may be added.

Adding stabilizers however may be problematic as those chemicals may have a negative impact on the complexation of the radionuclide into the chelating agent or may have a limited solubility and precipitate from the solution. Ethanol has been reported as stabilizer against radiolysis (WO 2008/009444*).* While ethanol might not have a negative impact on the complexation or a solubility issue, higher amounts of ethanol in an infusion solution may be physiologically problematic and may have a negative impact on the tolerability of the drug product.

Producing the drug product in high dilution has the disadvantage that large volumes of infusion solutions need to be administered to patients. For the convenience of patients and for drug tolerability reasons it would be highly desirable to provide the radiopharmaceutical drug product in a high concentration. Those highly concentrated solutions however are in particular prone to radiolysis. Therefore, there are contradictory positions between, on the one hand, avoiding radiolysis by dilution of the drug product but, on the other hand, avoiding patient discomfort during treatment by providing a concentrated drug solution. In Mathur et al. Cancer Biotherapy and Radiopharmaceuticals, 2017, 32(7), 266-273 a product of high concentration has been reported and claimed as being ready-to-use. However, that composition may be problematic with respect to tolerability as it contains high amounts of ethanol.

It remains therefore a challenge to design a ready-to-use radiopharmaceutical drug product which can be produced at commercial scale and delivered as a sufficiently stable and sterile solution in a high concentration which leads to a patient-convenient small infusion volume and which has a composition of high physiological tolerability (e.g. a composition which does not contain ethanol).

Myrna Luna-Gutiérrez et al: "Freeze-dried multi-dose kits for the fast preparation of 177Lu-Tyr3-octreotide and 177Lu-PSMA(inhibitor) under GMP conditions", J. Radioanal Nucl Chem, 314, 2181-2188 (2017), describes ¹⁷⁷Lu-Tyr³-octreotide and ¹⁷⁷Lu-PSMA(inhibitor) radiopeptides obtained from lyophilized formulations after reconstitution with sterile solutions of ¹⁷⁷LuCl₃ (40 GBq/mL), without the need for further purification or sterilization processes.

M.F. de Barboza et al: "Production and quality control of 177Lu-DOTATATE [177Lu-DOTA-Tyr3]-Octreotate: Clinical Application"*,* describes the production and quality control of ¹⁷⁷Lu-Tyr3-octreotate (¹⁷⁷Lu-DOTATATE), using DOTA (1,4,7,10-tetrazacyclododecane-N,N',N",N"'-tetra acetic acid) as chelating agent.

The EMA Assessment Report for Lutathera describes the evaluation of Lutathera, authorized by the EMA via the centralized procedure, and includes product information.

### SUMMARY OF THE INVENTION

The present inventors have now found a way to design and produce a highly concentrated radionuclide complex solution which is chemically and radiochemically very stable even if stored at ambient or short term elevated temperatures so that it can be produced on commercial scale and supplied as a ready-to-use radiopharmaceutical product.

The present invention is defined by the appended set of claims. In particular, the present invention provides:
A process for manufacturing a pharmaceutical aqueous solution,
wherein the pharmaceutical aqueous solution comprises:
   (a) a complex formed by
      (ai) the radionuclide ¹⁷⁷Lu (Lutetium-177), and
      (aii) DOTA-TATE; and
   (b) at least two different stabilizers against radiolytic degradation, wherein said component (b) comprises the stabilizers
      (bi) gentisic acid or a salt thereof, wherein the gentisic acid is present in a concentration of from 0.5 to 1 mg/mL, and
      (bii) ascorbic acid or a salt thereof, wherein the ascorbic acid is present in a concentration of from 2.0 to 5.0 mg/mL,
   wherein:
   said stabilizers are present in a total concentration of from 0.2 to 20.0 mg/mL,
   said solution has less than 1% ethanol by weight (w/w%),
   the radiochemical purity (determined by HPLC) is maintained at ≥ 95% for at least 72 h when stored at 25 °C,
   the pharmaceutical aqueous solution provides a radioactive dose of 7.4 GBq, and
   the volume of the pharmaceutical aqueous solution is 20.5 to 25.0 mL,
and wherein the process comprises the process steps:
   (1) Forming a complex of the radionuclide ¹⁷⁷Lu and DOTA-TATE by
      (1.1) preparing an aqueous solution comprising the radionuclide;
      (1.2) preparing an aqueous solution comprising the DOTA-TATE and at least one stabilizer against radiolytic degradation, wherein the at least one stabilizer comprises gentisic acid; and
      (1.3) mixing the solutions obtained in steps (1.1) and (1.2) and heating the resulting mixture;
   (2) Diluting the complex solution obtained by step (1) by
      (2.1) preparing an aqueous dilution solution comprising at least one stabilizer against radiolytic degradation, wherein the at least one stabilizer comprises ascorbic acid; and
      (2.2) mixing the complex solution obtained by step (1) with the dilution solution obtained by the step (2.1) to obtain the final solution.

Preferably, said stabilizers, component (b), are present in a total concentration of at least 2.7 mg/mL.

The present invention provides the following advantages:
The high concentration allows administering a high dose within a short time frame. The high dose of 7.4 GBq is provided in a small volume of 20.5 to 25.0 mL which allows the IV infusion administration to be completed within about 20 to 30 minutes.

The use of suitable stabilizers, according to the present invention as described, herein ensures high stability, at least 95%, 96%, 97%, 98%, 99% or 100% chemical stability with respect to the chemical purity for the cell receptor-binding molecule after 72 hours at 25 °C, even if this molecule is a sensitive peptide molecule. For DOTA-TATE, 100% chemical purity were found after 72 hours at 25 °C and even after 48 hours at 32 °C were found. Even under short term elevated temperature conditions (32°C for 12 h and 25° for 60 h) such high stability was found with respect to chemical purity.

Further, the use of suitable stabilizers, according to the present invention as described, herein ensures high stability, at least 95% radiochemical stability with respect to the radiochemical purity radionuclide complex. For ¹⁷⁷Lu-DOTA-TATE, at least 95% radiochemical purity were found after 72 hours at 25 °C. Even under short term elevated
temperature conditions (32°C for 12 h and 25° for 60 h) such high stability was found with respect to radiochemical purity.

While sufficient stability may be achieved already with one single stabilizer, the use of two stabilizers has been found to be of particular suitability in stabilizing sensitive radiopharmaceutical solutions. In particular, the presence of one stabilizer during complex formation and another stabilizer added after the complex formation is of advantage as it ensures that already during the complexation reaction, the cell receptor-binding molecule is protected against radiolysis and the other stabilizer enhances the protecting effect for the shelf-life period.

Further, by this sequential application of the two stabilizers it is ensured, that during complexation only a relatively small amount of stabilizer is present (which minimizes the potential interference of that stabilizer with the complexation reaction) and after complexation a large amount of a stabilizer combination is present (which strengthens the protective power of the stabilizers for the following drug product storage time period).

This sequential application of two stabilizers also reduces the overall thermal stress of those stabilizers as one of them is not present when the complexation reaction, which involves high temperatures, takes place.

Further, particularly the use of two different stabilizers is advantageous as this combination is more efficacious in reacting to the various different radicals possibly formed by the radiolysis of the cell receptor binding molecule than only one single stabilizer can do.

The composition of the radiopharmaceutical solution does not require the presence of ethanol. The solution is sufficiently stable without ethanol. The absence of ethanol is of advantage with respect to the physiological tolerability of the solution.

A shelf-life of at least 3 days is required to allow a radiopharmaceutical drug product to be manufactured from a centralized pharmaceutical production site and to commercialize it as a ready-to-use drug product.

Therefore, due to the high stability (72 h at 25°C) the present invention allows centralized pharmaceutical production at highest quality standards (e.g. cGMP) and at industrial scale, e.g. at 74 GBq or 148 GBq batch size which provides the drug product in numerous dose units, e.g. enough dose units for the treatment of 10 to 20 patients at the same time.

Further, due to the high stability, there is sufficient time for the present invention to be shipped from a centralized pharmaceutical production site to remote clinical centers.

Even further, due to the high stability, the present invention can be provided as a ready-to-use infusion solution which can be immediately administered to the patient without a need for the clinical staff to perform any preparatory work before administration.

The present invention relates to the very sensitive somatostatin analogue octreotate which is in particular prone to degradation reactions. Further, the present invention relates to the radionuclide Lutetium-177 with its specific radioactivity characteristics.

### DETAILED DESCRIPTION OF THE INVENTION

Herein after, the present invention is described in further detail and is exemplified.

In general, the present invention is concerned about a process for manufacturing a radiopharmaceutical aqueous solution. The solution is for intravenous (IV) use/application/administration. The solution is stable, concentrated, and ready-to-use.

The stability of the solution ascertained by the use of stabilizers against radiolytic degradation.

In general, the stabilizers used in accordance with the present inventions may be selected from gentisic acid (2,5-dihydroxybenzoic acid) or salts thereof, ascorbic acid (L-ascorbic acid, vitamin C) or salts thereof (e.g. sodium ascorbate), methionine, histidine, melatonine, ethanol, and Se-methionine. Preferred stabilizers are selected from gentisic acid or salts thereof and ascorbic acid or salts thereof.

Ethanol is considered as less preferred stabilizer due to tolerability issues associated with it if present in higher concentrations. Ethanol should be ideally avoided in the solutions of the present invention (in other words: free of ethanol).

According to the invention the amount of ethanol is less than 1 % by weight (w/w%) in the final solution which is foreseen to be injected/infused. Even more preferably, the solution is free of ethanol.

The "complex formed by" may be alternatively worded: "complex of".

The "different" in "two different stabilizers" refers to a difference in the chemical entity of such stabilizers. "Two different stabilizers" has the meaning that the two stabilizers are different chemical entities, e.g. gentisic acid and ascorbic acid are two different stabilizers.
"at least two" means two or more, however, preferably that just two stabilizers are present (not three or more). It is further preferred that ethanol is not one of the two stabilizers.

The herein indicated pH values are the pH values of the final solution. However, it also the pH during manufacturing of the solution, e.g. the pH during the complex formation.

"Shelf life" has herein its general meaning in the context of pharmaceutical products. The shelf life is the length of time that a pharmaceutical product may be stored while its product characteristics still comply with the product specification as defined during drug development and agreed by health authorities.

The present invention further provides the pharmaceutical aqueous solution as defined herein for use in the treatment of neuroendocrine tumors (NET).

Alternatively, the present invention provides the pharmaceutical aqueous solution of the invention for use in a method for the treatment of NET in human patients in need of such treatment which comprises administering an effective amount of the pharmaceutical aqueous solution as defined herein.

Neuroendocrine tumors (NET) which may be treated by the pharmaceutical aqueous solutions as defined here alone or in combinations in accordance with the present invention are selected from the group consisting of gastroenteropancreatic neuroendocrine tumor, carcinoid tumor, pheochromocytoma, paraganglioma, medullary thyroid cancer, pulmonary neuroendocrine tumor, thymic neuroendocrine tumor, a carcinoid tumor or a pancreatic neuroendocrine tumor, pituitary adenoma, adrenal gland tumors, Merkel cell carcinoma, breast cancer, Non-Hodgkin lymphoma, Hodgkin lymphoma, Head & Neck tumor, urothelial carcinoma (bladder), Renal Cell Carcinoma, Hepatocellular Carcinoma, GIST, neuroblastoma, bile duct tumor, cervix tumor, Ewing sarcoma, osteosarcoma, small cell lung cancer (SCLC), prostate cancer, melanoma, meningioma, glioma, medulloblastoma, hemangioblastoma, supratentorial primitive, neuroectodermal tumor, and esthesioneuroblastoma.

Further NET tumors which may be treated by the pharmaceutical aqueous solutions as defined here alone or in combinations in accordance with the present invention may be selected from the group consisting of functional carcinoid tumor, insulinoma, gastrinoma, vasoactive intestinal peptide (VIP) oma, glucagonoma, serotoninoma, histaminoma, ACTHoma, pheocromocytoma, and somatostatinoma.

The present invention further provides the pharmaceutical aqueous solution of the invention for use in combination or in combination therapy together with one or more therapeutic agents as outlined in the following:
In certain instances, other anti-cancer agents, anti-allergic agents, anti-nausea agents (or anti-emetics), pain relievers, cytoprotective agents, and combinations thereof.

General Chemotherapeutic agents considered for use in combination therapies include anastrozole (Arimidex^{®}), bicalutamide (Casodex^{®}), bleomycin sulfate (Blenoxane^{®}), busulfan (Myleran^{®}), busulfan injection (Busulfex^{®}), capecitabine (Xeloda^{®}), N4-pentoxycarbonyl-5-deoxy-5-fluorocytidine, carboplatin (Paraplatin^{®}), carmustine (BiCNU^{®}), chlorambucil (Leukeran^{®}), cisplatin (Platinol^{®}), cladribine (Leustatin^{®}), cyclophosphamide (Cytoxan^{®} or Neosar^{®}), cytarabine, cytosine arabinoside (Cytosar-U^{®}), cytarabine liposome injection (DepoCyt^{®}), dacarbazine (DTIC-Dome^{®}), dactinomycin (Actinomycin D, Cosmegan), daunorubicin hydrochloride (Cerubidine^{®}), daunorubicin citrate liposome injection (DaunoXome^{®}), dexamethasone, docetaxel (Taxotere^{®}), doxorubicin hydrochloride (Adriamycin^{®}, Rubex^{®}), etoposide (Vepesid^{®}), fludarabine phosphate (Fludara^{®}), 5-fluorouracil (Adrucil^{®}, Efudex^{®}), flutamide (Eulexin^{®}), tezacitibine, Gemcitabine (difluorodeoxycitidine), hydroxyurea (Hydrea^{®}), Idarubicin (Idamycin^{®}), ifosfamide (IFEX^{®}), irinotecan (Camptosar^{®}), L-asparaginase (ELSPAR^{®}), leucovorin calcium, melphalan (Alkeran^{®}), 6-mercaptopurine (Purinethol^{®}), methotrexate (Folex^{®}), mitoxantrone (Novantrone^{®}), mylotarg, paclitaxel (Taxol^{®}), nab-paclitaxel (Abraxane^{®}), phoenix (Yttrium90/MX-DTPA), pentostatin, polifeprosan 20 with carmustine implant (Gliadel^{®}), tamoxifen citrate (Nolvadex^{®}), teniposide (Vumon^{®}), 6-thioguanine, thiotepa, tirapazamine (Tirazone^{®}), topotecan hydrochloride for injection (Hycamptin^{®}), vinblastine (Velban^{®}), vincristine (Oncovin^{®}), and vinorelbine (Navelbine^{®}).

Anti-cancer agents of particular interest for combinations with the pharmaceutical aqueous solution of the present invention include:
***Tyrosine kinase inhibitors:*** Erlotinib hydrochloride (Tarceva^{®}); Linifanib (N-[4-(3-amino-1H-indazol-4-yl)phenyl]-N'-(2-fluoro-5-methylphenyl)urea, also known as ABT 869, available from Genentech); Sunitinib malate (Sutent^{®}); Bosutinib (4-[(2,4-dichloro-5-methoxyphenyl)amino]-6-methoxy-7-[3-(4-methylpiperazin-1-yl)propoxy]quinoline-3-carbonitrile, also known as SKI-606, and described in US Patent No. 6,780,996); Dasatinib (Sprycel^{®}); Pazopanib (Votrient^{®}); Sorafenib (Nexavar^{®}); Zactima (ZD6474); and Imatinib or Imatinib mesylate (Gilvec^{®} and Gleevec^{®}).
***Vascular Endothelial Growth Factor (VEGF) receptor inhibitors:*** Bevacizumab (Avastin^{®}), axitinib (Inlyta^{®}); Brivanib alaninate (BMS-582664, (*S*)-((*R*)-1-(4-(4-Fluoro-2-methyl-1*H*-indol-5-yloxy)-5-methylpyrrolo[2,1-*f*][1,2,4]triazin-6-yloxy)propan-2-yl)2-aminopropanoate); Sorafenib (Nexavar^{®}); Pazopanib (Votrient^{®}); Sunitinib malate (Sutent^{®}); Cediranib (AZD2171, CAS 288383-20-1); Vargatef (BIBF1120, CAS 928326-83-4); Foretinib (GSK1363089); Telatinib (BAY57-9352, CAS 332012-40-5); Apatinib (YN968D1, CAS 811803-05-1); Imatinib (Gleevec^{®}); Ponatinib (AP24534, CAS 943319-70-8); Tivozanib (AV951, CAS 475108-18-0); Regorafenib (BAY73-4506, CAS 755037-03-7); Vatalanib dihydrochloride (PTK787, CAS 212141-51-0); Brivanib (BMS-540215, CAS 649735-46-6); Vandetanib (Caprelsa^{®} or AZD6474); Motesanib diphosphate (AMG706, CAS 857876-30-3, N-(2,3-dihydro-3,3-dimethyl-1H-indol-6-yl)-2-[(4-pyridinylmethyl)amino]-3-pyridinecarboxamide, described in PCT Publication No. WO 02/066470); Dovitinib dilactic acid (TKI258, CAS 852433-84-2); Linfanib (ABT869, CAS 796967-16-3); Cabozantinib (XL184, CAS 849217-68-1); Lestaurtinib (CAS 111358-88-4); N-[5-[[[5-(1,1-Dimethylethyl)-2-oxazolyl]methyl]thio]-2-thiazolyl]-4-piperidinecarboxamide (BMS38703, CAS 345627-80-7); (3R,4R)-4-Amino-1-((4-((3-methoxyphenyl)amino)pyrrolo[2,1-f][1,2,4]triazin-5-yl)methyl)piperidin-3-ol (BMS690514); *N*-(3,4-Dichloro-2-fluorophenyl)-6-methoxy-7-[[(3aα,5β,6aα)-octahydro-2-methylcyclopenta[*c*]pyrrol-5-yl]methoxy]- 4-quinazolinamine (XL647, CAS 781613-23-8); 4-Methyl-3-[[1-methyl-6-(3-pyridinyl)-1*H*-pyrazolo[3,4-d]pyrimidin-4-yl]amino]-*N*-[3-(trifluoromethyl)phenyl]-benzamide (BHG712, CAS 940310-85-0); . and Aflibercept (Eylea^{®}), sulfatinib, surufatinib.
***Platelet-derived Growth Factor (PDGF) receptor inhibitors:*** Imatinib (Gleevec^{®}); Linifanib (N-[4-(3-amino-1H-indazol-4-yl)phenyl]-N'-(2-fluoro-5-methylphenyl)urea, also known as ABT 869, available from Genentech); Sunitinib malate (Sutent^{®}); Quizartinib (AC220, CAS 950769-58-1); Pazopanib (Votrient^{®}); Axitinib (Inlyta^{®}); Sorafenib (Nexavar^{®}); Vargatef (BIBF1120, CAS 928326-83-4); Telatinib (BAY57-9352, CAS 332012-40-5); Vatalanib dihydrochloride (PTK787, CAS 212141-51-0); and Motesanib diphosphate (AMG706, CAS 857876-30-3, N-(2,3-dihydro-3,3-dimethyl-1H-indol-6-yl)-2-[(4-pyridinylmethyl)amino]-3-pyridinecarboxamide, described in PCT Publication No. WO 02/066470).
***Fibroblast Growth Factor Receptor (FGFR) Inhibitors:*** Brivanib alaninate (BMS-582664, (*S*)-((*R*)-1-(4-(4-Fluoro-2-methyl-1*H*-indol-5-yloxy)-5-methylpyrrolo[2,1-*f*][1,2,4]triazin-6-yloxy)propan-2-yl)2-aminopropanoate); Vargatef (BIBF1120, CAS 928326-83-4); Dovitinib dilactic acid (TKI258, CAS 852433-84-2); 3-(2,6-Dichloro-3,5-dimethoxy-phenyl)-1-{6-[4-(4-ethyl-piperazin-1-yl)-phenylamino]-pyrimidin-4-yl}-1-methyl-urea (BGJ398, CAS 872511-34-7); Danusertib (PHA-739358); and N-[2-[[4-(Diethylamino)butyl]amino]-6-(3,5-dimethoxyphenyl)pyrido[2,3-d]pyrimidin-7-yl]-N'-(1,1-dimethylethyl)-urea (PD173074, CAS 219580-11-7). sulfatinib, surufatinib.
***Aurora kinase inhibitors:*** Danusertib (PHA-739358); *N*-[4-[[6-Methoxy-7-[3-(4-morpholinyl)propoxy]-4-quinazolinyl]amino]phenyl]benzamide (ZM447439, CAS 331771-20-1); 4-(2-Amino-4 -methyl-5-thiazolyl)-N-[4-(4-morpholinyl)phenyl]-2-pyrimidinamine (CYC116, CAS 693228-63-6); Tozasertib (VX680 or MK-0457, CAS 639089-54-6); Alisertib (MLN8237); (N-{2-[6-(4-Cyclobutylamino-5-trifluoromethyl-pyrimidine-2-ylamino)-(1S,4R)-1,2,3,4-tetrahydro-1,4-epiazano-naphthalen-9-yl]-2-oxo-ethyl}-acetamide) (PF-03814735); 4-[[9-Chloro-7-(2,6-difluorophenyl)-5H-pyrimido[5,4-*d*][2]benzazepin-2-yl]amino]-benzoic acid (MLN8054, CAS 869363-13-3); Cenisertib (R-763); Barasertib (AZD1152); and N-cyclopropyl-N'-[3-[6-(4-morpholinylmethyl)-1H-benzimidazol-2-yl]-1H-pyrazol-4-yl]-urea (AT9283).
***Cyclin-Dependent Kinase (CDK) inhibitors:*** Aloisine A; Alvocidib (also known as flavopiridol or HMR-1275, 2-(2-chlorophenyl)-5,7-dihydroxy-8-[(3S,4R)-3-hydroxy-1-methyl-4-piperidinyl]-4-chromenone, and described in US Patent No. 5,621,002); Crizotinib (PF-02341066, CAS 877399-52-5); 2-(2-Chlorophenyl)-5,7-dihydroxy-8-[(2*R*,3*S*)-2-(hydroxymethyl)-1-methyl-3-pyrrolidinyl]- 4*H*-1-benzopyran-4-one, hydrochloride (P276-00, CAS 920113-03-7); Indisulam (E7070); Roscovitine (CYC202); 6-Acetyl-8-cyclopentyl-5-methyl-2-(5-piperazin-1-yl-pyridin-2-ylamino)-8*H*-pyrido[2,3-*d*]pyrimidin-7-one, hydrochloride (PD0332991); Dinaciclib (SCH727965); N-[5-[[(5-*tert*-Butyloxazol-2-yl)methyl]thio]thiazol-2-yl]piperidine-4-carboxamide (BMS 387032, CAS 345627-80-7); 4-[[9-Chloro-7-(2,6-difluorophenyl)-5*H*-pyrimido[5,4-*d*][2]benzazepin-2-yl]amino]-benzoic acid (MLN8054, CAS 869363-13-3); 5-[3-(4,6-Difluoro-1H-benzimidazol-2-yl)-1H-indazol-5-yl]-N-ethyl-4-methyl-3-pyridinemethanamine (AG-024322, CAS 837364-57-5); 4-(2,6-Dichlorobenzoylamino)-1H-pyrazole-3-carboxylic acid N-(piperidin-4-yl)amide (AT7519, CAS 844442-38-2); 4-[2-Methyl-1-(1-methylethyl)-1H-imidazol-5-yl]-*N*-[4-(methylsulfonyl)phenyl]- 2-pyrimidinamine (AZD5438,CAS 602306-29-6); Palbociclib (PD-0332991); and (2R,3R)-3-[[2-[[3-[[S(R)]-S-cyclopropylsulfonimidoyl]-phenyl]amino]-5-(trifluoromethyl)-4-pyrimidinyl]oxy]-2-butanol (BAY 10000394), ribociclib.
***Checkpoint Kinase (CHK) inhibitors:*** 7-Hydroxystaurosporine (UCN-01); 6-Bromo-3-(1-methyl-1*H*-pyrazol-4-yl)-5-(3*R*)-3-piperidinyl-pyrazolo[1,5-a]pyrimidin-7-amine (SCH900776, CAS 891494-63-6); 5-(3-Fluorophenyl)-3-ureidothiophene-2-carboxylic acid N-[(S)-piperidin-3-yl]amide (AZD7762, CAS 860352-01-8); 4-[((3S)-1-Azabicyclo[2.2.2]oct-3-yl)amino]-3-(1H-benzimidazol-2-yl)-6-chloroquinolin-2(1H)-one (CHIR 124, CAS 405168-58-3); 7-Aminodactinomycin (7-AAD), Isogranulatimide, debromohymenialdisine; N-[5-Bromo-4-methyl-2-[(2S)-2-morpholinylmethoxy]-phenyl]-N'-(5-methyl-2-pyrazinyl)urea (LY2603618, CAS 911222-45-2); Sulforaphane (CAS 4478-93-7, 4-Methylsulfinylbutyl isothiocyanate); 9,10,11,12-Tetrahydro- 9,12-epoxy-1*H*-diindolo[1,2,3-*fg*:3',2',1'-*kl*]pyrrolo[3,4-*i*][1,6]benzodiazocine-1,3(2h)-dione (SB-218078, CAS 135897-06-2); and TAT-S216A (YGRKKRRQRRRLYRSPAMPENL), and CBP501 ((d-Bpa)sws(d-Phe-F5)(d-Cha)rrrqrr); and (αR)-α-amino-N-[5,6-dihydro-2-(1-methyl-1H-pyrazol-4-yl)-6-oxo-1H-pyrrolo[4,3,2-ef][2,3]benzodiazepin-8-yl]-Cyclohexaneacetamide (PF-0477736).
***3-Phosphoinositide-dependent kinase-1 (PDK1 or PDPK1) inhibitors:*** 7-2-Amino-*N*-[4-[5-(2-phenanthrenyl)-3-(trifluoromethyl)-1*H*-pyrazol-1-yl]phenyl]-acetamide (OSU-03012, CAS 742112-33-0); Pyrrolidine-1-carboxylic acid (3-{5-bromo-4-[2-(1H-imidazol-4-yl)-ethylamino]-pyrimidin-2-ylamino}-phenyl)-amide (BX912, CAS 702674-56-4); and 4-Dodecyl-*N*-1,3,4-thiadiazol-2-yl-benzenesulfonamide (PHT-427, CAS 1191951-57-1). ***Protein Kinase C (PKC) activators:*** Bryostatin I (bryo-1) and Sotrastaurin (AEB071). ***B-RAF inhibitors:*** Regorafenib (BAY73-4506, CAS 755037-03-7); Tuvizanib (AV951, CAS 475108-18-0); Vemurafenib (Zelboraf^{®}, PLX-4032, CAS 918504-65-1); 5-[1-(2-Hydroxyethyl)-3-(pyridin-4-yl)-1H-pyrazol-4-yl]-2,3-dihydroinden-1-one oxime (GDC-0879, CAS 905281-76-7); 5-[2-[4-[2-(Dimethylamino)ethoxy]phenyl]-5-(4-pyridinyl)-1*H*-imidazol-4-yl]-2,3-dihydro-1*H*-Inden-1-one oxime (GSK2118436 or SB590885); (+/-)-Methyl (5-(2-(5-chloro-2-methylphenyl)-1-hydroxy-3-oxo-2,3-dihydro-1H-isoindol-1-yl)-1H-benzimidazol-2-yl)carbamate (also known as XL-281 and BMS908662) and N-(3-(5-chloro-1H-pyrrolo[2,3-b]pyridine-3-carbonyl)-2,4-difluorophenyl)propane-1-sulfonamide (also known as PLX4720).
***C-RAF Inhibitors:*** Sorafenib (Nexavar^{®}); 3-(Dimethylamino)-*N*-[3-[(4-hydroxybenzoyl)amino]-4-methylphenyl]-benzamide (ZM336372, CAS 208260-29-1); and 3-(1-cyano-1-methylethyl)-*N*-[3-[(3,4-dihydro-3-methyl-4-oxo-6-quinazolinyl)amino]-4-methylphenyl]-benzamide (AZ628, CAS 1007871-84-2).
***Human Granulocyte colony-stimulating factor (G-CSF) modulators:*** Filgrastim (Neupogen^{®}); Sunitinib malate (Sutent^{®}); Pegilgrastim (Neulasta^{®}) and Quizartinib (AC220, CAS 950769-58-1).
***RET Inhibitors:*** Sunitinib malate (Sutent^{®}); Vandetanib (Caprelsa^{®}); Motesanib diphosphate (AMG706, CAS 857876-30-3, N-(2,3-dihydro-3,3-dimethyl-1H-indol-6-yl)-2-[(4-pyridinylmethyl)amino]-3-pyridinecarboxamide, described in PCT Publication No. WO 02/066470); Sorafenib (BAY 43-9006); Regorafenib (BAY73-4506, CAS 755037-03-7); and Danusertib (PHA-739358).
***FMS-like Tyrosine kinase 3 (FLT3) Inhibitors or CD135:*** Sunitinib malate (Sutent^{®}); Quizartinib (AC220, CAS 950769-58-1); *N*-[(1-Methyl-4-piperidinyl)methyl]-3-[3-(trifluoromethoxy)phenyl]- Imidazo[1,2-*b*]pyridazin-6-amine sulfate (SGI-1776, CAS 1173928-26-1); and Vargatef (BIBF1120, CAS 928326-83-4).
***c-KIT Inhibitors:*** Pazopanib (Votrient^{®}); Dovitinib dilactic acid (TKI258, CAS 852433-84-2); Motesanib diphosphate (AMG706, CAS 857876-30-3, N-(2,3-dihydro-3,3-dimethyl-1H-indol-6-yl)-2-[(4-pyridinylmethyl)amino]-3-pyridinecarboxamide, described in PCT Publication No. WO 02/066470); Masitinib (Masivet^{®}); Regorafenib (BAY73-4506, CAS 755037-03-7); Tivozanib (AV951, CAS 475108-18-0); Vatalanib dihydrochloride (PTK787, CAS 212141-51-0); Telatinib (BAY57-9352, CAS 332012-40-5); Foretinib (GSK1363089, formerly XL880, CAS 849217-64-7); Sunitinib malate (Sutent^{®}); Quizartinib (AC220, CAS 950769-58-1); Axitinib (Inlyta^{®}); Dasatinib (BMS-345825); and Sorafenib (Nexavar^{®}).
***Bcr*/*Abl kinase inhibitors:*** Imatinib (Gleevec^{®}); Inilotinib hydrochloride; Nilotinib (Tasigna^{®}); Dasatinib (BMS-345825); Bosutinib (SKI-606); Ponatinib (AP24534); Bafetinib (INNO406); Danusertib (PHA-739358), AT9283 (CAS 1133385-83-7); Saracatinib (AZD0530); and *N*-[2-[(1S,4R)-6-[[4-(Cyclobutylamino)-5-(trifluoromethyl)-2-pyrimidinyl]amino]-1,2,3,4-tetrahydronaphthalen-1,4-imin-9-yl]-2-oxoethyl]-acetamide (PF-03814735, CAS 942487-16-3).
***IGF-1R inhibitors:*** Linsitnib (OSI-906); [7-[*trans*-3-[(Azetidin-1-yl)methyl]cyclobutyl]-5-(3-benzyloxyphenyl)-7H-pyrrolo[2,3-d]pyrimidin-4-yl]amine (AEW541, CAS 475488-34-7); [5-(3-Benzyloxyphenyl)-7-[*trans*-3-[(pyrrolidin-1-yl)methyl]cyclobutyl]-7H-pyrrolo[2,3-d]pyrimidin-4-yl]amine (ADW742 or GSK552602A, CAS 475488-23-4); (2-[[3-Bromo-5-(1,1-dimethylethyl)-4-hydroxyphenyl]methylene]-propanedinitrile (Tyrphostin AG1024, CAS 65678-07-1); 4-[[(2*S*)-2-(3-Chlorophenyl)-2-hydroxyethyl]amino]-3-[7-methyl-5-(4-morpholinyl)-1*H-*benzimidazol-2-yl]- 2(1*H*)-pyridinone (BMS536924, CAS 468740-43-4); 4-[2-[4-[[(2*S*)-2-(3-Chlorophenyl)-2-hydroxyethyl]amino]-1,2-dihydro-2-oxo-3-pyridinyl]-7-methyl-1*H-*benzimidazol-5-yl]- 1-piperazinepropanenitrile (BMS554417, CAS 468741-42-6); (2*S*)-1-[4-[(5-Cyclopropyl-1*H*-pyrazol-3-yl)amino]pyrrolo[2,1-*f*][1,2,4]triazin-2-yl]-*N*-(6-fluoro-3-pyridinyl)-2-methyl-2-pyrrolidinecarboxamide (BMS754807, CAS 1001350-96-4); Picropodophyllotoxin (AXL1717); and Nordihydroguareacetic acid.
IGF-1R antibodies: Figitumumab (CP751871); Cixutumumab (IMC-A12); Ganitumab (AMG-479); Robatumumab (SCH-717454); Dalotuzumab (MK0646); R1507 (available from Roche); BIIB022 (available from Biogen); and MEDI-573 (available from MedImmune). ***MET inhibitors:*** Cabozantinib (XL184, CAS 849217-68-1); Foretinib (GSK1363089, formerly XL880, CAS 849217-64-7); Tivantinib (ARQ197, CAS 1000873-98-2); 1-(2-Hydroxy-2-methylpropyl)-*N*-(5-(7-methoxyquinolin-4-yloxy)pyridin-2-yl)-5-methyl-3-oxo-2-phenyl-2,3-dihydro-1*H*-pyrazole-4-carboxamide (AMG 458); Cryzotinib (Xalkori^{®}, PF-02341066); (3Z)-5-(2,3-Dihydro-1H-indol-1-ylsulfonyl)-3-({3,5-dimethyl-4-[(4-methylpiperazin-1-yl)carbonyl]-1H-pyrrol-2-yl}methylene)-1,3-dihydro-2H-indol-2-one (SU11271); (3Z)-N-(3-Chlorophenyl)-3-({3,5-dimethyl-4-[(4-methylpiperazin-1-yl)carbonyl]-1H-pyrrol-2-yl}methylene)-N-methyl-2-oxoindoline-5-sulfonamide (SU11274); (3Z)-N-(3-Chlorophenyl)-3-{[3,5-dimethyl-4-(3-morpholin-4-ylpropyl)-1H-pyrrol-2-yl]methylene}-N-methyl-2-oxoindoline-5-sulfonamide (SU11606); 6-[Difluoro[6-(1-methyl-1H-pyrazol-4-yl)-1,2,4-triazolo[4,3-b]pyridazin-3-yl]methyl]-quinoline (JNJ38877605, CAS 943540-75-8); 2-[4-[1-(Quinolin-6-ylmethyl)-1H-[1,2,3]triazolo[4,5-b]pyrazin-6-yl]-1H-pyrazol-1-yl]ethanol (PF04217903, CAS 956905-27-4); N-((2R)-1,4-Dioxan-2-ylmethyl)-N-methyl-N'-[3-(1-methyl-1H-pyrazol-4-yl)-5-oxo-5H-benzo[4,5]cyclohepta[1,2-b]pyridin-7-yl]sulfamide (MK2461, CAS 917879-39-1); 6-[[6-(1-Methyl-1*H*-pyrazol-4-yl)-1,2,4-triazolo[4,3-*b*]pyridazin-3-yl]thio]-quinoline (SGX523, CAS 1022150-57-7); and (3Z)-5-[[(2,6-Dichlorophenyl)methyl]sulfonyl]-3-[[3,5-dimethyl-4-[[(2*R*)-2-(1-pyrrolidinylmethyl)-1-pyrrolidinyl]carbonyl]-1*H*-pyrrol-2-yl]methylene]-1,3-dihydro-2H-indol-2-one (PHA665752, CAS 477575-56-7).
***Epidermal growth factor receptor (EGFR) inhibitors:*** Erlotinib hydrochloride (Tarceva^{®}), Gefitnib (Iressa^{®}); N-[4-[(3-Chloro-4-fluorophenyl)amino]-7-[[(3"S")-tetrahydro-3-furanyl]oxy]-6-quinazolinyl]-4(dimethylamino)-2-butenamide, Tovok^{®}); Vandetanib (Caprelsa^{®}); Lapatinib (Tykerb^{®}); (3R,4R)-4-Amino-1-((4-((3-methoxyphenyl)amino)pyrrolo[2,1-f][1,2,4]triazin-5-yl)methyl)piperidin-3-ol (BMS690514); Canertinib dihydrochloride (CI-1033); 6-[4-[(4-Ethyl-1-piperazinyl)methyl]phenyl]-*N*-[(1*R*)-1-phenylethyl]- 7*H*-Pyrrolo[2,3-d]pyrimidin-4-amine (AEE788, CAS 497839-62-0); Mubritinib (TAK165); Pelitinib (EKB569); Afatinib (BIBW2992); Neratinib (HKI-272); *N*-[4-[[1-[(3-Fluorophenyl)methyl]-1*H*-indazol-5-yl]amino]-5-methylpyrrolo[2,1-*f*][1,2,4]triazin-6-yl]-carbamic acid, (3*S*)-3-morpholinylmethyl ester (BMS599626); *N*-(3,4-Dichloro-2-fluorophenyl)-6-methoxy-7-[[(3aα,5β,6aα)-octahydro-2-methylcyclopenta[*c*]pyrrol-5-yl]methoxy]- 4-quinazolinamine (XL647, CAS 781613-23-8); and 4-[4-[[(1*R*)-1-Phenylethyl]amino]-7*H*-pyrrolo[2,3-d]pyrimidin-6-yl]-phenol (PKI166, CAS 187724-61-4). *EGFR antibodies:* Cetuximab (Erbitux^{®}); Panitumumab (Vectibix^{®}); Matuzumab (EMD-72000); Trastuzumab (Herceptin^{®}); Nimotuzumab (hR3); Zalutumumab; TheraCIM h-R3; MDX0447 (CAS 339151-96-1); and ch806 (mAb-806, CAS 946414-09-1).
***mTOR inhibitors:*** Temsirolimus (Torisel^{®}); Ridaforolimus (formally known as deferolimus, (1*R*,2*R*,4*S*)-4-[(2*R*)-2 [(1*R*,9*S*,12*S*,15*R*,16*E*,18*R*,19*R*,21*R*, 23*S*,24*E*,26*E*,28*Z*,30*S*,32*S*,35*R*)-1,18-dihydroxy-19,30-dimethoxy-15,17,21,23, 29,35-hexamethyl-2,3,10,14,20-pentaoxo-11,36-dioxa-4-azatricyclo[30.3.1.0^{4,9}] hexatriaconta-16,24,26,28-tetraen-12-yl]propyl]-2-methoxycyclohexyl dimethylphosphinate, also known as AP23573 and MK8669, and described in PCT Publication No. WO 03/064383); Everolimus (Afinitor^{®} or RAD001); Rapamycin (AY22989, Sirolimus^{®}); Simapimod (CAS 164301-51-3); (5-{2,4-Bis[(3*S*)-3-methylmorpholin-4-yl]pyrido[2,3-d]pyrimidin-7-yl}-2-methoxyphenyl)methanol (AZD8055); 2-Amino-8-[*trans*-4-(2-hydroxyethoxy)cyclohexyl]-6-(6-methoxy-3-pyridinyl)-4-methyl-pyrido[2,3-*d*]pyrimidin-7(8H)-one (PF04691502, CAS 1013101-36-4); *N*²-[1,4-dioxo-4-[[4-(4-oxo-8-phenyl-4*H*-1-benzopyran-2-yl)morpholinium-4-yl]methoxy]butyl]-L-arginylglycyl-L-α-aspartylL-serine-, inner salt (SF1126, CAS 936487-67-1); and N-[4-[[[3-[(3,5-dimethoxyphenyl)amino]-2-quinoxalinyl]amino]sulfonyl]phenyl]-3-methoxy-4-methyl-benzamide (XL765, also known as SAR245409); and (1r,4r)-4-(4-amino-5-(7-methoxy-1H-indol-2-yl)imidazo[1,5-f][1,2,4]triazin-7-yl)cyclohexanecarboxylic acid (OSI-027).
**Mitogen-activated protein kinase *(MEK) inhibitors:*** XL-518 (also known as GDC-0973, Cas No. 1029872-29-4, available from ACC Corp.); Selumetinib (5-[(4-bromo-2-chlorophenyl)amino]-4-fluoro-N-(2-hydroxyethoxy)-1-methyl-1H-benzimidazole-6-carboxamide, also known as AZD6244 or ARRY 142886, described in PCT Publication No. WO2003077914); 2-[(2-Chloro-4-iodophenyl)amino]-N-(cyclopropylmethoxy)-3,4-difluoro-benzamide (also known as CI-1040 or PD184352 and described in PCT Publication No. WO2000035436); N-[(2R)-2,3-Dihydroxypropoxy]-3,4-difluoro-2-[(2-fluoro-4-iodophenyl)amino]- benzamide (also known as PD0325901 and described in PCT Publication No. WO2002006213); 2,3-Bis[amino[(2-aminophenyl)thio]methylene]-butanedinitrile (also known as U0126 and described in US Patent No. 2,779,780); N-[3,4-Difluoro-2-[(2-fluoro-4-iodophenyl)amino]-6-methoxyphenyl]-1-[(2R)-2,3-dihydroxypropyl]-cyclopropanesulfonamide (also known as RDEA119 or BAY869766 and described in PCT Publication No. WO2007014011); (3S,4R,5Z,8S,9S,11E)-14-(Ethylamino)-8,9,16-trihydroxy-3,4-dimethyl-3,4,9, 19-tetrahydro-1H-2-benzoxacyclotetradecine-1,7(8H)-dione] (also known as E6201 and described in PCT Publication No. WO2003076424); 2'-Amino-3'-methoxyflavone (also known as PD98059 available from Biaffin GmbH & Co., KG, Germany); Vemurafenib (PLX-4032, CAS 918504-65-1); (R)-3-(2,3-Dihydroxypropyl)-6-fluoro-5-(2-fluoro-4-iodophenylamino)-8-methylpyrido[2,3-d]pyrimidine-4,7(3H,8H)-dione (TAK-733, CAS 1035555-63-5); Pimasertib (AS-703026, CAS 1204531-26-9); Trametinib dimethyl sulfoxide (GSK-1120212, CAS 1204531-25-80); 2-(2-Fluoro-4-iodophenylamino)-N-(2-hydroxyethoxy)-1,5-dimethyl-6-oxo-1,6-dihydropyridine-3-carboxamide (AZD 8330); and 3,4-Difluoro-2-[(2-fluoro-4-iodophenyl)amino]-N-(2-hydroxyethoxy)-5-[(3-oxo-[1,2]oxazinan-2-yl) methyl]benzamide (CH 4987655 or Ro 4987655).
***Alkylating agents:*** Oxaliplatin (Eloxatin^{®}); Temozolomide (Temodar^{®} and Temodal^{®}); Dactinomycin (also known as actinomycin-D, Cosmegen^{®}); Melphalan (also known as L-PAM, L-sarcolysin, and phenylalanine mustard, Alkeran^{®}); Altretamine (also known as hexamethylmelamine (HMM), Hexalen^{®}); Carmustine (BiCNU^{®}); Bendamustine (Treanda^{®}); Busulfan (Busulfex^{®} and Myleran^{®}); Carboplatin (Paraplatin^{®}); Lomustine (also known as CCNU, CeeNU^{®}); Cisplatin (also known as COOP, Platinol^{®} and Platinol^{®}-AQ); Chlorambucil (Leukeran^{®}); Cyclophosphamide (Cytoxan^{®} and Neosar^{®}); Dacarbazine (also known as DTIC, DIC and imidazole carboxamide, DTIC-Dome^{®}); Altretamine (also known as hexamethylmelamine (HMM), Hexalen^{®}); Ifosfamide (Ifex^{®}); Prednumustine; Procarbazine (Matulane^{®}); Mechlorethamine (also known as nitrogen mustard, mustine and mechloroethamine hydrochloride, Mustargen^{®}); Streptozocin (Zanosar^{®}); Thiotepa (also known as thiophosphoamide, TESPA and TSPA, Thioplex^{®}); Cyclophosphamide (Endoxan^{®}, Cytoxan^{®}, Neosar^{®}, Procytox^{®}, Revimmune^{®}); and Bendamustine HCl (Treanda^{®}).
***Aromatase inhibitors:*** Exemestane (Aromasin^{®}); Letrozole (Femara^{®}); and Anastrozole (Arimidex^{®}).
***Topoisomerase I inhibitors:*** Irinotecan (Camptosar^{®}); Topotecan hydrochloride (Hycamtin^{®}); and 7-Ethyl-10-hydroxycampothecin (SN38).
***Topoisomerase II inhibitors:*** Etoposide (VP-16 and Etoposide phosphate, Toposar^{®}, VePesid^{®} and Etopophos^{®}); Teniposide (VM-26, Vumon^{®}); and Tafluposide .
***DNA Synthesis inhibitors:*** Capecitabine (Xeloda^{®}); Gemcitabine hydrochloride (Gemzar^{®}); Nelarabine ((2*R*,3*S*,4*R*,5*R*)-2-(2-amino-6-methoxy-purin-9-yl)-5-(hydroxymethyl)oxolane-3,4-diol, Arranon^{®} and Atriance^{®}); and Sapacitabine (1-(2-cyano-2-deoxy-β-D-arabinofuranosyl)-4-(palmitoylamino)pyrimidin-2(1*H*)-one).
***Folate Antagonists or Antifolates:*** Trimetrexate glucuronate (Neutrexin^{®}); Piritrexim isethionate (BW201U); Pemetrexed (LY231514); Raltitrexed (Tomudex^{®}); and Methotrexate (Rheumatrex^{®}, Trexal^{®}).
***Immunomodulators:*** Afutuzumab (available from Roche^{®}); Pegfilgrastim (Neulasta^{®}); Lenalidomide (CC-5013, Revlimid^{®}); Thalidomide (Thalomid^{®}), Actimid (CC4047); and IRX-2 (mixture of human cytokines including interleukin 1, interleukin 2, and interferon γ, CAS 951209-71-5, available from IRX Therapeutics).
***G-Protein-coupled Somatostain receptors Inhibitors:*** Octreotide (also known as octreotide acetate, Sandostatin^{®} and Sandostatin LAR^{®}); Lanreotide acetate (CAS 127984-74-1); Seglitide (MK678); Vapreotide acetate (Sanvar^{®}); and Cyclo(D-Trp-Lys-Abu-Phe-MeAla-Tyr)( BIM23027).
***Interleukin-11 and Synthetic Interleukin-11 (IL-11):*** Oprelvekin (Neumega^{®}). ***Erythropoietin and Synthetic erythropoietin:*** Erythropoietin (Epogen^{®} and Procrit^{®}); Darbepoetin alfa (Aranesp^{®}); Peginesatide (Hematide^{®}); and EPO covalently linked to polyethylene glycol (Micera^{®}).
***Histone deacetylase (HDAC) inhibitors:*** Voninostat (Zolinza^{®}); Romidepsin (Istodax^{®}); Treichostatin A (TSA); Oxamflatin; Vorinostat (Zolinza^{®}, Suberoylanilide hydroxamic acid); Pyroxamide (syberoyl-3-aminopyridineamide hydroxamic acid); Trapoxin A (RF-1023A); Trapoxin B (RF-10238); Cyclo[(α*S*,2*S*)-α-amino-η-oxo-2-oxiraneoctanoyl-*O*-methyl-D-tyrosyl-L-isoleucyl-L-prolyl] (Cyl-1); Cyclo[(α*S*,2*S*)-α-amino-η-oxo-2-oxiraneoctanoyl-*O-*methyl-D-tyrosyl-L-isoleucyl-(2*S*)-2-piperidinecarbonyl] (Cyl-2); Cyclic[L-alanyl-D-alanyl-(2S)-η-oxo-L-α-aminooxiraneoctanoyl-D-prolyl] (HC-toxin); Cyclo[(α*S*,2*S*)-α-amino-η-oxo-2-oxiraneoctanoyl-D-phenylalanyl-L-leucyl-(2*S*)-2-piperidinecarbonyl] (WF-3161); Chlamydocin ((S)-Cyclic(2-methylalanyl-L-phenylalanyl-D-prolyl-η-oxo-L-α-aminooxiraneoctanoyl); Apicidin (Cyclo(8-oxo-L-2-aminodecanoyl-1-methoxy-L-tryptophyl-L-isoleucyl-D-2-piperidinecarbonyl); Romidepsin (Istodax^{®}, FR-901228); 4-Phenylbutyrate; Spiruchostatin A; Mylproin (Valproic acid); Entinostat (MS-275, N-(2-Aminophenyl)-4-[N-(pyridine-3-yl-methoxycarbonyl)-amino-methyl]-benzamide); and Depudecin (4,5:8,9-dianhydro-1,2,6,7,11-pentadeoxy- D-*threo*-D-*ido*-Undeca-1,6-dienitol).
***Biologic response modifiers:*** Include therapeutics such as interferons, interleukins, colony-stimulating factors, monoclonal antibodies, vaccines (therapeutic and prophylactic), gene therapy, and nonspecific immunomodulating agents. Interferon alpha (Intron^{®}, Roferson^{®}-A); Interferon beta; Interferon gamma; Interleukin-2 (IL-2 or aldesleukin, Proleukin^{®}); Filgrastim (Neupogen^{®}); Sargramostim (Leukine^{®}); Erythropoietin (epoetin); Interleukin-11 (oprelvekin); Imiquimod (Aldara^{®}); Lenalidomide (Revlimid^{®}); Rituximab (Rituxan^{®}); Trastuzumab (Herceptin^{®}); Bacillus calmette-guerin (theraCys^{®} and TICE^{®} BCG); Levamisole (Ergamisol^{®}); and Denileukin diftitox (Ontak^{®}).
***Plant Alkaloids:*** Paclitaxel (Taxol and Onxal^{™}); Paclitaxel protein-bound (Abraxane^{®}); Vinblastine (also known as vinblastine sulfate, vincaleukoblastine and VLB, Alkaban-AQ^{®} and Velban^{®}); Vincristine (also known as vincristine sulfate, LCR, and VCR, Oncovin^{®} and Vincasar Pfs^{®}); and Vinorelbine (Navelbine^{®}).
***Taxane anti-neoplastic agents:*** Paclitaxel (Taxol^{®}); Docetaxel (Taxotere^{®}); Cabazitaxel (Jevtana^{®}, 1-hydroxy-7β,10β-dimethoxy-9-oxo-5β,20-epoxytax-11-ene-2α,4,13α-triyl-4-acetate-2-benzoate-13-[(2R,3S)-3-{[(tert-butoxy)carbonyl]amino}-2-hydroxy-3-phenylpropanoate); and Larotaxel ((2α,3ξ,4α,5β,7α,10β,13α)-4,10-bis(acetyloxy)-13-({(2*R*,3*S*)-3- [(*tert*-butoxycarbonyl) amino]-2-hydroxy-3-phenylpropanoyl}oxy)-1- hydroxy-9-oxo-5,20-epoxy-7,19-cyclotax-11-en-2-yl benzoate).
***Heat Shock Protein (HSP) inhibitors:*** Tanespimycin (17-allylamino-17-demethoxygeldanamycin, also known as KOS-953 and 17-AAG, available from SIGMA, and described in US Patent No. 4,261,989); Retaspimycin (IPI504), Ganetespib (STA-9090); [6-Chloro-9-(4-methoxy-3,5-dimethylpyridin-2-ylmethyl)-9H-purin-2-yl]amine (BIIB021 or CNF2024, CAS 848695-25-0); *trans*-4-[[2-(Aminocarbonyl)-5-[4,5,6,7-tetrahydro-6,6-dimethyl-4-oxo-3-(trifluoromethyl)-1*H*-indazol-1-yl]phenyl]amino]cyclohexyl glycine ester (SNX5422 or PF04929113, CAS 908115-27-5); and 17-Dimethylaminoethylamino-17-demethoxygeldanamycin (17-DMAG).
***Thrombopoietin (TpoR) agonists:*** Eltrombopag (SB497115, Promacta^{®} and Revolade^{®}); and Romiplostim (Nplate^{®}).
***Demethylating agents:*** 5-Azacitidine (Vidaza^{®}); and Decitabine (Dacogen^{®}).
***Cytokines:*** Interleukin-2 (also known as aldesleukin and IL-2, Proleukin^{®}); Interleukin-11 (also known as oprevelkin, Neumega^{®}); and Alpha interferon alfa (also known as IFN-alpha, Intron^{®} A, and Roferon-A^{®}).
***17 α-hydroxylase*/*C17,20 lyase (CYP17A1) inhibitors:*** Abiraterone acetate (Zyitga^{®}).
***Miscellaneous cytotoxic agents:*** Arsenic trioxide (Trisenox^{®}); Asparaginase (also known as L-asparaginase, Erwinia L-asparaginase, Elspar^{®} and Kidrolase^{®}); and Asparaginase Erwinia Chrysanthemi (Erwinaze^{®}).
***C-C Chemokine receptor 4 (CCR4) Antibody:*** Mogamulizumab (Potelligent^{®})
***CD20 antibodies:*** Rituximab (Riuxan^{®} and MabThera^{®}); and Tositumomab (Bexxar^{®}); and Ofatumumab (Arzerra^{®}).
***CD20 Antibody Drug Conjugates:*** Ibritumomab tiuxetan (Zevalin^{®}); and Tositumomab,
***CD22 Antibody Drug Conjugates:*** Inotuzumab ozogamicin (also referred to as CMC-544 and WAY-207294, available from Hangzhou Sage Chemical Co., Ltd.)
***CD30 mAb-cytotoxin Conjugates:*** Brentuximab vedotin (Adcetrix^{®});
***CD33 Antibody Drug Conjugates:*** Gemtuzumab ozogamicin (Mylotarg^{®}),
***CD40 antibodies:*** Dacetuzumab (also known as SGN-40 or huS2C6, available from Seattle Genetics, Inc),
***CD52 antibodies:*** Alemtuzumab (Campath^{®}),
***Anti-CS1 antibodies:*** Elotuzumab (HuLuc63, CAS No. 915296-00-3)
***CTLA-4 inhibitor antibodies:*** Tremelimumab (IgG2 monoclonal antibody available from Pfizer, formerly known as ticilimumab, CP-675,206); and Ipilimumab (CTLA-4 antibody, also known as MDX-010, CAS No. 477202-00-9).
**TPH inhibitors:** telotristat
**PARP (poly ADP ribose polymerase) inhibitors:** olaparib (Lynparza), rucaparib (Rubraca), Niraparib (Zeluja), Talazoparib, Veliparib.
**PD-1 Inhibitors** : Spartalizumab (PDR001, Novartis), Nivolumab (Bristol-Myers Squibb), Pembrolizumab (Merck & Co), Pidilizumab (CureTech), MEDI0680 (Medimmune), REGN2810 (Regeneron), TSR-042 (Tesaro), PF-06801591 (Pfizer), BGB-A317 (Beigene), BGB-108 (Beigene), INCSHR1210 (Incyte), or AMP-224 (Amplimmune).
**PD-L1 inhibitors:** Durvalumab, Atezolizumab, Avelumab

In an embodiment, the one or more therapeutic agents are selected from the group consisting of octreotide, lanreotide, vaproreotide, pasireotide, satoreotide, everolimus, temozolomide, telotristat, sunitinib, sulfatinib, ribociclib, entinostat, and pazopanib. In particular embodiments, those combinations are for use in the treatment of NET tumors, e.g. GEP-NET, pulmonary NET, pNET, lung NET, Carcinoid syndrome, SCLC.

In particular embodiments, the one or more therapeutics agents, are one or more immuno-oncology therapeutic agents selected from the group consisting of PD-1, PD-L1 and CTLA-4 inhibitors, in particular the I-O therapeutic agents selected from Spartalizumab, Nivolumab, Pembrolizumab, Pidilizumab, Durvalumab, Atezolizumab, Avelumab, Ipilimumab, and Tremelimumab. In particular embodiments, those combinations are for use in the treatment of NET tumors, e.g. GEP-NET, pulmonary NET, pNET, lung NET, Carcinoid syndrome, SCLC.

### DEFINITIONS

In the following, terms as used herein are defined in their meaning.

The term "about" or "ca." has herein the meaning that the following value may vary for ± 20%, preferably ± 10%, more preferably ± 5%, even more preferably ± 2%, even more preferably ± 1%.

Unless otherwise defined, "%" has herein the meaning of weight percent (wt%), also refered to as weight by weight percent (w/w%).
"total concentration": sum of one or more individual concentrations.
"aqueous solution": a solution of one or more solute in water.
"complex formed by
   (ai) a radionuclide, and
   (aii) a cell receptor binding organic moiety linked to a chelating agent":
      The radionuclide metal ion is forming a non-covalent bond with the functional groups of the chelating agent, e.g. amines or carboxylic acids. The chelating agent has at least two such complexing functional groups to be able to form a chelate complex.
      "linked": the somatostatin receptor binding peptide is either directly linked to the chelating agent or connected via a linker molecule, preferably it is directly linked. The linking bond(s) is (are) either covalent or non-covalent bond(s) between the cell receptor binding organic moiety (and the linker) and the chelating agent, preferably the bond(s) is (are) covalent.
      "Stabilizer against radiolytic degradation": stabilizing agent which protects organic molecules against radiolytic degradation, e.g. when a gamma ray emitted from the radionuclide is cleaving a bond between the atoms of an organic molecules and radicals are formed, those radicals are then scavenged by the stabilizer which avoids the radicals undergoing any other chemical reactions which might lead to undesired, potentially ineffective or even toxic molecules. Therefore, those stabilizers are also referred to as "free radical scavengers" or in short "radical scavengers". Other alternative terms for those stabilizers are "radiation stability enhancers", "radiolytic stabilizers", or simply "quenchers".
      "stabilizer(s) is (are) present in the solution during the complex formation of components (ai) and (aii)": first stabilizer present and optionally also second stabilizer present, i.e. either first stabilizer alone or in combination with second stabilizer present.
      "present during the complex formation": stabilizer(s) are in either the radionuclide solution or in the chelating agent containing solution before those two solutions are added and potentially elevated temperatures are applied to facilitate the complex formation. Preferably the stabilizer(s) are in the chelating agent containing solution.
      "only the first stabilizer is present during the complex formation of components (ai) and (aii)": the first stabilizer is present, the second is not present. In other words only one stabilizer is present.
      "second stabilizer is added after the complex formation of components (ai) and (aii)": Regardless of whether the second stabilizers may have been present already during
         the complex formation or not, the second stabilizer is added after the complex forming reaction is completed, e.g. after the reacting solution which might have been heated up to an elevated temperature is again cooled down to ambient temperature.

The cell receptor binding moiety and the chelating agent form together the following molecule:
DOTA-TATE: [DOTA⁰,D-Phe¹,Tyr³]octreotate, DOTA-Tyr³-Octreotate, DOTA-d-Phe-Cys-Tyr-d-Trp-Lys-Thr-Cys-Thr (cyclo 2,7), oxodotreotide (INN), represented by the following formula :

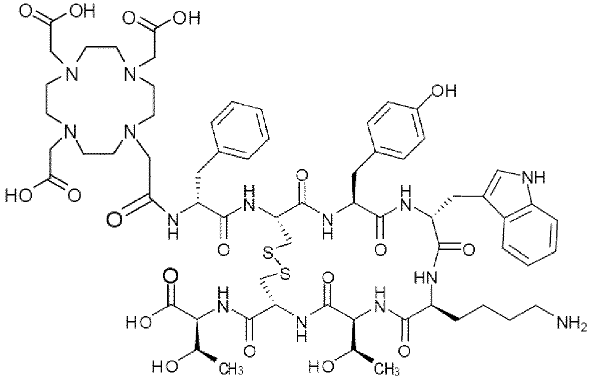

The "cell receptor binding moiety linked to the chelating agent" molecule for the present invention is DOTA-TATE.

For the present invention, the complex formed by (or the complex of) the radionuclide and the cell receptor binding moiety linked to the chelating agent is ¹⁷⁷Lu-DOTA-TATE, which is also referred to as Lutetium (177Lu) oxodotreotide (INN), i.e. hydrogen [N-{[4,7,10-tris(carboxylato-κ*O*-methyl)-1,4,7,10-tetraazacyclododecan-1-yl-κ⁴N¹,N⁴,N⁷,N¹⁰]acetyl-κ*O*}-D-phenylalanyl-L-cysteinyl-tyrosyl-D-tryptophyl-L-lysyl-L-threonyl-L-cysteinyl-L-threoninato cyclic (2→7)-disulfide(4-)](177Lu)lutetate(1-)
and is represented by the following formulas:

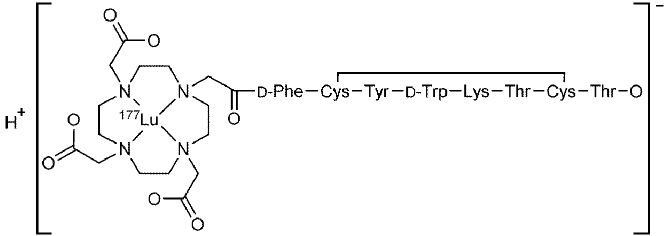

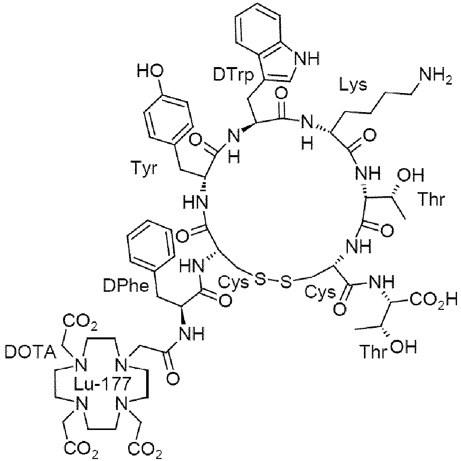

"Buffer for a pH from 4.5 to 6.0": may be an acetate buffer, citrate buffer (e.g. citrate + HCl or citric acid + Disodium hydrogenphosphate) or phosphate buffer (e.g. Sodium dihydrogenphosphate + Disodium hydrogenphosphate), preferably said buffer is an acetate buffer, preferably said acetate buffer is composed of acetic acid and sodium acetate.

"Sequestering agent", a chelating agent suitable to complex the radionuclide metal ions, preferably DTPA: Diethylenetriaminepentaacetic acid.

"for commercial use": the drug product, e.g. a pharmaceutical aqueous solution, is able to obtain (preferably has obtained) marketing authorization by health authorities, e.g. US-FDA or EMA, by complying with all drug product quality and stability requirements as demanded by such health authorities, is able to be manufactured (preferably is manufactured) from or at a pharmaceutical production site at commercial scale followed by a quality control testing procedure, and is able to be supplied (preferably is supplied) to remotely located end users, e.g. hospitals or patients.

"Combination": refers to either a fixed combination in one dosage unit form, or a combined administration where a compound of the present invention and a combination partner (e.g. another drug as explained below, also referred to as "therapeutic agent" or "co-agent") may be administered independently at the same time or separately within time intervals, especially where these time intervals allow that the combination partners show a cooperative, e.g. synergistic effect. The single components may be packaged in a kit or separately. One or both of the components (e.g., powders or liquids) may be reconstituted or diluted to a desired dose prior to administration. The terms "coadministration" or "combined administration" or the like as utilized herein are meant to encompass administration of the selected combination partner to a single subject in need thereof (e.g. a patient), and are intended to include treatment regimens in which the agents are not necessarily administered by the same route of administration or at the same time. The term "pharmaceutical combination" as used herein means a product that results from the mixing or combining of more than one therapeutic agent and includes both fixed and non-fixed combinations of the therapeutic agents. The term "fixed combination" means that the therapeutic agents, e.g. a compound of the present invention and a combination partner, are both administered to a patient simultaneously in the form of a single entity or dosage. The term "non-fixed combination" means that the therapeutic agents, e.g. a compound of the present invention and a combination partner, are both administered to a patient as separate entities either simultaneously, concurrently or sequentially with no specific time limits, wherein such administration provides therapeutically effective levels of the two compounds in the body of the patient. The latter also applies to cocktail therapy, e.g. the administration of three or more therapeutic agent.

### EXAMPLES

Hereinafter, the present invention is described in more details and specifically with reference to the examples, which however are not intended to limit the present invention.

Materials:
The ¹⁷⁷LuCl₃ may be obtained from commercial sources, e.g. I.D.B. Holland BV. The DOTA⁰-Tyr³-Octreotate may be obtained from commercial sources, e.g. by piCHEM Forschungs-und Entwicklungs GmbH, Austria. All other components of the drug product are commercially available from various sources.

### Example 1: Composition of drug product

The Drug Product (¹⁷⁷Lu-DOTA⁰-Tyr³-Octreotate 370 MBq/mL solution for infusion) is designed as a sterile ready-to-use solution for infusion containing ¹⁷⁷Lu-DOTA⁰-Tyr³-Octreotate as Drug Substance with a volumetric activity of 370 MBq/mL at reference date and time (calibration time (tc)). Calibration time (tc) corresponds to the End of Production (EOP = t0) which is the time of measurement of the activity of the first QC vial. The shelf-life of Drug Product is defined as 72 hours after calibration time. Drug Product is a single dose vial, containing suitable amount of solution that allows delivery of 7.4 GBq of radioactivity at injection time.

### Manufacturing site prepares single doses calibrated within the range of 7.4 GBq ± 10 %

(200 mCi) after the end of production. Certificates of analysis reports both the exact activity provided and the time when this activity is reached. This value is declared as "Injection time: {DD MM YYYY} {hh:mm} UTC". Considering the variable injection time and constant decay of the radionuclide, the filling volume needed for an activity of 7.4 GBq at injection time is calculated and can range from 20.5 and 25.0 mL.

### Composition of drug product per mL

| **Property/Component** | **Quantity (Unit/mL)** | **Function** |
|---|---|---|
| ¹⁷⁷Lu-DOTA⁰-Tyr³-Octreotate (volumetric activity) | 370 MBq/mL at t_{c} (EOP) | Drug Substance |
| X-DOTA⁰-Tyr³-Octreotate | 10 µg/mL | Total peptide content |
| Specific Activity (GBq/Total peptide) | ≥ 53 GBq/µmol at EOP | NA |

| Excipients | | |
|---|---|---|
| Acetic acid | 0.48 mg/mL | pH adjuster |
| Sodium acetate | 0.66 mg/mL | pH adjuster |
| Gentisic acid | 0.63 mg/mL | RSE |
| Ascorbic acid | 2.80 mg/mL | RSE |
| DTPA | 0.05 mg/mL | Sequestering agent |
| Sodium chloride (NaCl) | 6.85 mg/mL | Isotonizing agent |
| Sodium hydroxide (NaOH) | 0.64 mg/mL | pH adjuster |
| Water for injection | Ad 1 mL | Solvent |

| | | |
|---|---|---|
| *EOP: End of Production=t₀=activity measurement of the first vial=calibration time t_{c}* *RSE: Radiation Stability Enhancer* | | |

### Example 2: Manufacturing of drug product

For a 74 GBq batch size (2 Ci batch size) a ¹⁷⁷LuCl₃ solution, about 74 GBq in HCl, is mixed together with a DOTA-Tyr³-Octreotate (about 2 mg) solution, and a Reaction Buffer solution, containing an antioxidant agent (and stabilizer against radiolytic degradation) (i.e. Gentisic acid, about 157 mg) and a buffer system (i.e. Acetate buffer system), resulting in a total of about 5.5 mL solution, which is used for radiolabelling that occurs at a temperature of about 90 to about 98°C within less than 15 minutes.

The synthesis is carried out using a single use disposable kit cassette installed on the front of the synthesis module which contains the fluid pathway (tubing), reactor vial and sealed reagent vials.

The obtained mother solution is diluted with a solution containing a chelating agent (i.e. DTPA), an antioxidant agent (i.e. Ascorbic acid) sodium hydroxide, and sodium chloride and, then sterile filtered through 0.2 µm to give the ready-to-use solution as described in Example 1 with a pH of 4.5-6.0, in particular 5.2-5.3. Finally, the solution is dispensed in volumes of from 20.5 to 25.0 mL into sterile vials. The stoppered vials are enclosed within lead containers for protective shielding.

Manufacturing Process can also be implemented for batch sizes higher than 74GBq. In this case the amount of the raw materials (Lutetium, peptide and Reaction Buffer) are multiplied to guarantee the same raw materials ratio.

### Example 3: Stability study results after storage at various temperature conditions.

The following table provides the stability test data for a batch produced at 74 GBq batch size according to the process described in Example 2.

| ***Time points*** | | **t(0)** | **t(0+24h)** | **t(0+48h)** | **t(0+72h)** |
|---|---|---|---|---|---|
| ***Stability at 5* ± *2 °C*** | | ***CQ1*** | | | ***11 mL*** |
| | | | | | ***21.8 mL*** |
| pH | | 5.3 | n.d. | n.d. | 5.3 |
| | | | | | 5.3 |
| Chemical purity (RP-UV-HPLC) | Peptide purity (%) | 100.0 | n.d. | n.d. | 100.0 |
| | | | | | 100.0 |
| Radiochemical purity (RP-γβ-HPLC) | ¹⁷⁷Lu-DOTA⁰-Tyr³-octreotate (%) | 98.37 | n.d. | n.d. | 96.09 |
| | | | | | 96.40 |
| ***Stability at 25* ± *2 °C*** | | ***CQ1*** | ***5 mL*** | ***5mL*** | ***5 mL*** |
| | | | | | ***24.7 mL*** |
| pH | | 5.3 | 5.3 | 5.2 | 5.2 |
| | | | | | 5.3 |
| Chemical purity (RP-UV-HPLC) | Peptide purity (%) | 100.0 | 100.0 | 100.0 | 100.0 |
| Radiochemical purity (RP-γβ-HPLC) | ¹⁷⁷Lu-DOTA⁰-Tyr³-octreotate (%) | 98.28 | 96.99 | 96.29 | 95.02 |
| | | | | | 95.62 |

| ***Time points*** | | **t(0)** | **t(0+24h)** | **t(0+48h)** | **t(0+72h)** |
|---|---|---|---|---|---|
| ***Stability at 32*** ± ***2 °C*** | | ***CQ1*** | ***5.6 mL*** | ***5.6 mL*** | |
| | | | ***22.2 mL*** | ***22.2 mL*** | |
| pH | | 5.3 | n.d. | 5.3 | n.d. |
| | | | | 5.3 | |
| Chemical purity (RP-UV-HPLC) | Peptide purity (%) | 100.0 | 100.0 | 100.0 | n.d. |
| | | | 100.0 | 100.0 | |
| Radiochemical purity (RP-γβ-HPLC) | ¹⁷⁷Lu-DOTA⁰-Tyr³-octreotate (%) | 98.37 | 96.03 | 94.45 | n.d. |
| | | | 96.51 | 95.45 | |

| ***Time points*** | | **t(0)** | **t(0+24h)** | **t(0+48h)** | **t(0+72h)** |
|---|---|---|---|---|---|
| ***Stability at 32* ± *2 °C per 12h and at 25* ± *2 °C per 60h*** | | ***CQ1*** | | | ***11 mL*** |
| Chemical purity (RP-UV-HPLC) | Peptide purity (%) | 100.0 | n.d. | n.d. | 100.0 |
| Radiochemical purity (RP-γβ-HPLC) | ¹⁷⁷Lu-DOTA⁰-Tyr³-octreotate (%) | 98.28 | n.d. | n.d. | 95.01 |

| | | | | | |
|---|---|---|---|---|---|
| "n.d." = not determined; "LOD" = limit of detection | | | | | |

Very similar good stability results were obtained for batches produced at 148 GBq batch size.

## Claims

1. A process for manufacturing a pharmaceutical aqueous solution,
wherein the pharmaceutical aqueous solution comprises:
(a) a complex formed by
(ai) the radionuclide ¹⁷⁷Lu (Lutetium-177), and
(aii) DOTA-TATE; and
(b) at least two different stabilizers against radiolytic degradation, wherein said component (b) comprises the stabilizers
(bi) gentisic acid or a salt thereof, wherein the gentisic acid is present in a concentration of from 0.5 to 1 mg/mL, and
(bii) ascorbic acid or a salt thereof, wherein the ascorbic acid is present in a concentration of from 2.0 to 5.0 mg/mL,
wherein:
said stabilizers are present in a total concentration of from 0.2 to 20.0 mg/mL,
said solution has less than 1% ethanol by weight (w/w%),
the radiochemical purity (determined by HPLC) is maintained at ≥ 95% for at least 72 h when stored at 25 °C,
the pharmaceutical aqueous solution provides a radioactive dose of 7.4 GBq, and
the volume of the pharmaceutical aqueous solution is 20.5 to 25.0 mL,
and wherein the process comprises the process steps:
(1) Forming a complex of the radionuclide ¹⁷⁷Lu and DOTA-TATE by
(1.1) preparing an aqueous solution comprising the radionuclide;
(1.2) preparing an aqueous solution comprising the DOTA-TATE and at least one stabilizer against radiolytic degradation, wherein the at least one stabilizer comprises gentisic acid; and
(1.3) mixing the solutions obtained in steps (1.1) and (1.2) and heating the resulting mixture;
(2) Diluting the complex solution obtained by step (1) by
(2.1) preparing an aqueous dilution solution comprising at least one stabilizer against radiolytic degradation, wherein the at least one stabilizer comprises ascorbic acid; and
(2.2) mixing the complex solution obtained by step (1) with the dilution solution obtained by the step (2.1) to obtain the final solution.

2. The process according to claim 1, wherein the pharmaceutical aqueous solution has a shelf life of at least 72 h when stored at ≤ 25 °C, in particular at least 72 h when stored at 25 °C.

3. The process according to any one of claims 1 to 2, wherein the pharmaceutical aqueous solution is ready-to-use.

4. The process according to any one of claims 1 to 3, wherein the solution prepared in step (1.2) comprises only one stabilizer which is gentisic acid and the solution prepared in step (2.1) comprises only one stabilizer which is ascorbic acid.

5. The process according to any one of claims 1 to 4, wherein the solution prepared in step (1.2) comprises stabilizer/stabilizers in a total concentration of from 15 to 50 mg/mL, preferably from 20 to 40 mg/mL.

6. The process according to any one of claims 1 to 4, wherein the solution prepared in step (1.2) comprises only one stabilizer which is gentisic acid in a concentration of from 20 to 40 mg/mL, preferably from 25 to 35 mg/mL.

7. The process according to any one of claims 1 to 6, wherein in step (1.3) the resulting mixture is heated to a temperature of from 70 to 99 °C, preferably from 90 to 98 °C, for from 2 to 59 min, preferably from 10 to 15 min.

8. The process according to any one of claims 1 to 7, wherein the pharmaceutical aqueous solution further comprises:
(c) diethylenetriaminepentaacetic acid (DTPA) or a salt thereof in a concentration of from 0.01 to 0.10 mg/mL.

9. The process according to claim 8, wherein the solution of step (2.1) further comprises the DTPA or a salt thereof.

10. The process according to any one of claims 1 to 9, wherein the solution of step (1.1) comprises LuCl₃ and HCl.

11. The process according to any one of claims 1 to 10, wherein the solution of step (1.2) comprises ¹⁷⁷Lu-DOTA-TATE, gentisic acid, acetic acid, and sodium acetate.

12. The process according to any one of claims 1 to 11, wherein the solution of step (2.1) comprises DTPA, and ascorbic acid.

## Patentansprüche

1. Verfahren zur Herstellung einer pharmazeutischen wässrigen Lösung,
wobei die pharmazeutische wässrige Lösung Folgendes umfasst:
(a) einen Komplex, gebildet von
(ai) dem Radionuklid ¹⁷⁷Lu (Lutetium-177) und
(aii) DOTA-TATE; und
(b) mindestens zwei verschiedene Stabilisatoren gegen radiolytischen Abbau, wobei die Komponente (b) die Stabilisatoren
(bi) Gentisinsäure oder ein Salz davon, wobei die Gentisinsäure in einer Konzentration von 0,5 bis 1 mg/ml vorliegt, und
(bii) Ascorbinsäure oder ein Salz davon, wobei die Ascorbinsäure in einer Konzentration von 2,0 bis 5,0 mg/ml vorliegt,
umfasst, wobei:
die Stabilisatoren in einer Gesamtkonzentration von 0,2 bis 20,0 mg/ml vorliegen,
die Lösung weniger als 1 Gew.-% Ethanol aufweist,
die radiochemische Reinheit (bestimmt durch HPLC) bei Lagerung bei 25 °C mindestens 72 h bei ≥ 95 % gehalten wird,
die pharmazeutische wässrige Lösung eine radioaktive Dosis von 7,4 Gbq liefert und
das Volumen der pharmazeutischen wässrigen Lösung 20,5 bis 25,0 ml beträgt,
und wobei das Verfahren die folgenden Verfahrensschritte umfasst:
(1) Bilden eines Komplexes aus dem Radionuklid ¹⁷⁷Lu und DOTA-TATE durch
(1.1) Herstellen einer das Radionuklid umfassenden wässrigen Lösung;
(1.2) Herstellen einer das DOTA-TATE und mindestens einen Stabilisator gegen radiolytischen Abbau umfassenden wässrigen Lösung, wobei der mindestens eine Stabilisator Gentisinsäure umfasst; und
(1.3) Mischen der in Schritt (1.1) und Schritt (1.2) erhaltenen Lösungen und Erhitzen des resultierenden Gemischs;
(2) Verdünnen der durch Schritt (1) erhaltenen Komplexlösung durch
(2.1) Herstellen einer wässrigen Verdünnungslösung, die mindestens einen Stabilisator gegen radiolytischen Abbau umfasst, wobei der mindestens eine Stabilisator Ascorbinsäure umfasst; und
(2.2) Mischen der durch Schritt (1) erhaltenen Komplexlösung mit der durch den Schritt (2.1) erhaltenen Verdünnungslösung zum Erhalt der fertigen Lösung.

2. Verfahren nach Anspruch 1, wobei die pharmazeutische wässrige Lösung eine Lagerstabilität von mindestens 72 h bei Lagerung bei ≤ 25 °C, insbesondere mindestens 72 h bei Lagerung bei 25 °C, aufweist.

3. Verfahren nach einem der Ansprüche 1 bis 2, wobei die pharmazeutische wässrige Lösung gebrauchsfertig ist.

4. Verfahren nach einem der Ansprüche 1 bis 3, wobei die in Schritt (1.2) hergestellte Lösung nur einen Stabilisator umfasst, bei dem es sich um Gentisinsäure handelt, und die in Schritt (2.1) hergestellte Lösung nur einen Stabilisator umfasst, bei dem es sich um Ascorbinsäure handelt.

5. Verfahren nach einem der Ansprüche 1 bis 4, wobei die in Schritt (1.2) hergestellte Lösung Stabilisator/Stabilisatoren in einer Gesamtkonzentration von 15 bis 50 mg/ml, bevorzugt 20 bis 40 mg/ml, umfasst.

6. Verfahren nach einem der Ansprüche 1 bis 4, wobei die in Schritt (1.2) hergestellte Lösung nur einen Stabilisator umfasst, bei dem es sich um Gentisinsäure in einer Konzentration von 20 bis 40 mg/ml, bevorzugt 25 bis 35 mg/ml, handelt.

7. Verfahren nach einem der Ansprüche 1 bis 6, wobei in Schritt (1.3) das erhaltene Gemisch 2 bis 59 min, bevorzugt 10 bis 15 min, auf eine Temperatur von 70 bis 99 °C, bevorzugt 90 bis 98 °C, erhitzt wird.

8. Verfahren nach einem der Ansprüche 1 bis 7, wobei die pharmazeutische wässrige Lösung ferner Folgendes umfasst:
(c) Diethylentriaminpentaessigsäure (DTPA) oder ein Salz davon in einer Konzentration von 0,01 bis 0,10 mg/ml.

9. Verfahren nach Anspruch 8, wobei die Lösung von Schritt (2.1) ferner die DTPA oder ein Salz davon umfasst.

10. Verfahren nach einem der Ansprüche 1 bis 9, wobei die Lösung von Schritt (1.1) LuCl₃ und HCl umfasst.

11. Verfahren nach einem der Ansprüche 1 bis 10, wobei die Lösung von Schritt (1.2) ¹⁷⁷Lu-DOTA-TATE, Gentisinsäure, Essigsäure und Natriumacetat umfasst.

12. Verfahren nach einem der Ansprüche 1 bis 11, wobei die Lösung von Schritt (2.1) DTPA und Ascorbinsäure umfasst.

## Revendications

1. Procédé de fabrication d'une solution aqueuse pharmaceutique,
la solution aqueuse pharmaceutique comprenant :
(a) un complexe formé par
(ai) le radionucléide ¹⁷⁷Lu (lutétium-177), et
(aii) DOTA-TATE ; et
(b) au moins deux stabilisants différents contre la dégradation radiolytique, ledit composant (b) comprenant les stabilisants
(bi) acide gentisique ou un sel correspondant, l'acide gentisique étant présent en une concentration allant de 0,5 à 1 mg/ml, et
(bi) acide ascorbique ou un sel correspondant, l'acide ascorbique étant présent en une concentration allant de 2,0 à 5,0 mg/ml,
dans lequel :
lesdits stabilisants sont présents en une concentration totale allant de 0,2 à 20,0 mg/ml,
ladite solution contient moins de 1 % d'éthanol en poids (% p/p),
la pureté radiochimique (déterminée par HPLC) est maintenue à ≥ 95 % pendant au moins 72 h lorsqu'elle est conservée à 25 °C,
la solution aqueuse pharmaceutique fournit une dose radioactive de 7,4 GBq, et
le volume de la solution aqueuse pharmaceutique est de 20,5 à 25,0 ml,
et le procédé comprenant les étapes de procédé suivantes :
(1) formation d'un complexe du radionucléide ¹⁷⁷Lu et de DOTA-TATE par
(1.1) préparation d'une solution aqueuse comprenant le radionucléide ;
(1.2) préparation d'une solution aqueuse comprenant le DOTA-TATE et au moins un stabilisant contre la dégradation radiolytique, ledit stabilisant comprenant de l'acide gentisique ; et
(1.3) mélange des solutions obtenues aux étapes (1.1) et (1.2) et chauffage du mélange résultant ;
(2) dilution de la solution de complexe obtenue à l'étape (1) par
(2.1) préparation d'une solution aqueuse de dilution comprenant au moins un stabilisant contre la dégradation radiolytique, ledit stabilisant comprenant de l'acide ascorbique ; et
(2.2) mélange de la solution de complexe obtenue à l'étape (1) avec la solution de dilution obtenue à l'étape (2.1) pour obtenir la solution finale.

2. Procédé selon la revendication 1, la solution aqueuse pharmaceutique ayant une durée de conservation d'au moins 72 h lorsqu'elle est conservée à ≤ 25 °C, en particulier d'au moins 72 h lorsqu'elle est conservée à 25 °C.

3. Procédé selon l'une quelconque des revendications 1 à 2, la solution aqueuse pharmaceutique étant prête à l'emploi.

4. Procédé selon l'une quelconque des revendications 1 à 3, la solution préparée à l'étape (1.2) comprenant un seul stabilisant qui est l'acide gentisique, et la solution préparée à l'étape (2.1) comprenant un seul stabilisant qui est l'acide ascorbique.

5. Procédé selon l'une quelconque des revendications 1 à 4, la solution préparée à l'étape (1.2) comprenant un ou plusieurs stabilisants à une concentration totale de 15 à 50 mg/ml, de préférence de 20 à 40 mg/ml.

6. Procédé selon l'une quelconque des revendications 1 à 4, la solution préparée à l'étape (1.2) comprenant un seul stabilisant à une concentration de 20 à 40 mg/ml, de préférence de 25 à 35 mg/ml.

7. Procédé selon l'une quelconque des revendications 1 à 6, dans lequel, à l'étape (1.3), le mélange résultant est chauffé à une température de 70 à 99 °C, de préférence de 90 à 98 °C, pendant 2 à 59 min, de préférence de 10 à 15 min.

8. Procédé selon l'une quelconque des revendications 1 à 7, la solution aqueuse pharmaceutique comprenant en outre :
(c) de l'acide diéthylènetriaminepentaacétique (DTPA) ou un sel correspondant à une concentration de 0,01 à 0,10 mg/ml.

9. Procédé selon la revendication 8, la solution de l'étape (2.1) comprenant en outre du DTPA ou un sel correspondant.

10. Procédé selon l'une quelconque des revendications 1 à 9, la solution de l'étape (1.1) comprenant LuCl₃ et HCl.

11. Procédé selon l'une quelconque des revendications 1 à 10, la solution de l'étape (1.2) comprenant ¹⁷⁷Lu-DOTA-TATE, de l'acide gentisique, de l'acide acétique et de l'acétate de sodium.

12. Procédé selon l'une quelconque des revendications 1 à 11, la solution de l'étape (2.1) comprenant DTPA et de l'acide ascorbique.
